# FASCICULE DE BREVET EUROPEEN

(11) **EP 1 581 276 B1**
(45) Date de publication et mention de la délivrance du brevet: **16.11.2011**
(21) Numéro de dépôt: 03810017.8
(22) Date de dépôt: 23.12.2003
(51) Int. Cl.: A61L 27/46, A61K 6/033, A61K 6/083, A61L 27/44, A61L 2/025, A61L 2/18

(54) **MATÉRIAU À USAGE MÉDICAL OU VÉTÉRINAIRE, SON PROCÉDÉ D' OBTENTION ET SES APPLICATIONS**
MATERIAL ZUR MEDIZINISCHEN ODER TIERMEDIZINISCHEN VERWENDUNG UND VERFAHREN ZUR HERSTELLUNG
MEDICAL OR VETERINARY MATERIAL, METHOD FOR THE PRODUCTION AND USE THEREOF

(30) Priorité: 24.12.2002 FR 0216627
(43) Date de publication de la demande: 05.10.2005
(60) Demande divisionnaire: 10183852.2
(73) Titulaire: Cadorel, Catherine, 44380 Pornichet (FR)
(72) Inventeur: COUGOULIC, Jean-Pierre, F-44380 Pornichet (FR)
(74) Mandataire: Hamann, Jean-Christophe
(86) Numéro de dépôt international: PCT/FR2003/050208
(87) Numéro de publication internationale: WO 2004/058319

(56) Documents cités:
- WO-A-96/03161
- DE-A- 10 055 465
- BOYAN B D ET AL: "Role of material surfaces in regulating bone and cartilage cell response" BIOMATERIALS, ELSEVIER SCIENCE PUBLISHERS BV., BARKING, GB, vol. 17, no. 2, 1996, pages 137-146, XP004032811 ISSN: 0142-9612
- TAN K H ET AL: "Scaffold development using selective laser sintering of polyetheretherketone-hydroxyapatite biocomposite blends" BIOMATERIALS, ELSEVIER SCIENCE PUBLISHERS BV., BARKING, GB, vol. 24, no. 18, août 2003 (2003-08), pages 3115-3123, XP004441792 ISSN: 0142-9612
- ABU BAKAR M S ET AL: "Tensile properties, tension-tension fatigue and biological response of polyetheretherketone-hydroxyapatite composites for load-bearing orthopedic implants" BIOMATERIALS, ELSEVIER SCIENCE PUBLISHERS BV., BARKING, GB, vol. 24, no. 13, juin 2003 (2003-06), pages 2245-2250, XP004420014 ISSN: 0142-9612

## Description

La présente invention concerne un matériau original qui peut être utilisé dans le domaine médical ou vétérinaire, en particulier mais non exclusivement pour la réalisation d'implants endo-osseux, notamment dentaires, ou pour la réalisation de prothèses osseuses. L'invention concerne également le procédé d'obtention de ce matériau, ainsi que ses applications.

De nombreux types de matériaux, métalliques ou plastiques, sont utilisés dans le domaine médical ou vétérinaire pour le remplacement de structures biologiques (os en particulier) ou pour la fixation d'organes fonctionnels (implants dentaires ou autres...).

Le choix du matériau est réalisé en fonction de ses caractéristiques structurelles intrinsèques et également en fonction de sa biocompatibilité en terme de tolérance ou, ce qui est mieux, en terme d'acceptation biologique.

Le document FR-A-2 722 694 décrit un matériau moulé pour la réalisation d'implants endo-osseux ou de prothèses osseuses, constitué d'un polymère thermoplastique (en particulier du poly(étheréthercétone), encore appelé PEEK) comprenant de l'hydroxyapatite de calcium, du phosphate tricalcique, de l'acide orthophosphorique et une zéolite du type TiO₂.

Malgré les résultats encourageants obtenus avec ce type de matériau, il s'avère que les résultats en terme d'intégration biologique ne sont pas tout à fait satisfaisants.

La présente invention propose un nouveau matériau dérivé de celui décrit dans le document précité FR-A-2 722 694, qui combine de bonnes qualités mécaniques générales et une très bonne biocompatibilité en terme d'acceptation biologique, susceptible d'autoriser une utilisation efficace tant dans le domaine médical que le domaine vétérinaire.

Le matériau correspondant se présente sous la forme d'une pièce moulée constituée d'un liant biocompatible contenant un ou plusieurs composés réalisant un apport de calcium et de phosphore, laquelle pièce moulée a subi une opération de décapage surfacique.

Ce décapage assure la mise en surface et donc l'accès en surface des éléments ajoutés au liant, notamment du calcium et du phosphore ; cela permet la création ou tout au moins de favoriser la création de liaisons ioniques entre ces éléments ajoutés et les éléments chimiques environnants, minéraux ou organiques, après implantation biologique de la pièce de matière. Et en cas de présence d'éléments ajoutés résorbables, une fois ces éléments disparus, les tissus, les cellules biologiques ou les éléments chimiques environnants peuvent trouver une place d'intégration dans le matériau.

Cette particularité permet d'améliorer l'adhésion et la colonisation cellulaire en vue d'assurer une bonne acceptation biologique de type greffe et une bonne biocompatibilité de l'implant.

Le liant biocompatible est choisi en fonction de ses caractéristiques physiques après mise en forme notamment par une opération de moulage-injection. A titre d'exemple on peut utiliser un polymère thermoplastique du genre poly(étheréthercétone), un polyéther cétone, un polyéther block amides, un polysulfone, un polytétrafluoroéthylène ou encore un polyimide ; on peut aussi utiliser un polymère naturel, notamment du genre cellulose. Ce polymère peut être résorbable ou non.

Etant donné son haut module de Young et ses caractéristiques structurelles intéressantes qui se rapprochent de celles de l'os, on utilise de préférence le poly(étheréthercétone) (PEEK). Le PEEK est un polymère semi-cristallin formé d'une chaîne linéaire aromatique basée sur la répétition d'unités suivantes :

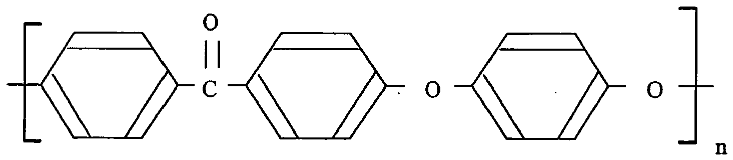

Les caractéristiques de ce polymère sont développées dans la brochure commerciale éditée en 1992 par la Société ICI MATERIALS : « Victrex PEEK, the high temperature engineering thermoplastic-properties and processing ».

Les apports en calcium et en phosphore sont avantageusement réalisés par des phosphates de calcium issus par exemple de phosphate tricalcique (Ca₃ (PO₄)₂), de phosphate dicalcique ou monétite (Ca H PO₄), d'hydroxyapatite de calcium ((Ca₅ (PO₄)₃ OH) ou (Ca₁₀ (PO₄)₆H20)), avec une formulation stoechiométrique ou non, ou de produits les contenant.

La présence de phosphates de calcium permet au matériau de se rapprocher de la composition naturelle de l'os afin de renforcer la biocompatibilité. On utilise de préférence des produits contenant des phosphates de calcium au moins partiellement résorbables.

En particulier, l'hydroxyapatite de calcium est un composant que l'on retrouve dans l'os. On peut avantageusement l'utiliser sous sa forme non stoechiométrique, car il est de ce fait légèrement résorbable, ce qui est intéressant pour l'intégration tissulaire.

Le phosphate dicalcique ou tricalcique présente l'intérêt d'être peu onéreux et d'être l'un des composants biologiques de base pour la formation d'hydroxyapatite de calcium ; il est d'autre part résorbable et a également une fonction cicatrisante. On peut encore utiliser ces différents apports de phosphates de calcium en mélange.

Outre l'apport de phosphates de calcium le matériau moulé conforme à l'invention peut comporter de l'acide orthophosphorique (H₃ (PO₄)). L'acide orthophosphorique en nature est prescrit comme fixateur de calcium et comme acidifiant ; il s'agit en outre d'un constituant fondamental des nucléotides qui sont les unités de base des acides nucléiques, lesquels participent à la constitution du noyau des cellules vivantes.

En outre, le matériau selon l'invention est avantageusement chargé d'un ou de plusieurs composés permettant de créer ou de favoriser les liaisons électrostatiques avec le milieu environnant. Cette ou ces charges peuvent être choisies parmi les zéolites et/ou certains oxydes ; on peut en particulier envisager d'utiliser les céramiques genre dioxyde de titane (TiO₂), dioxyde de zirconium (ZrO₂), oxyde d'aluminium (Al₂O₃) ou dioxyde de silicium (SiO₂).

Les charges en question sont des composés électrostatiques qui permettent une fonction d'accrochage ionique ; elles ont en outre une masse molaire élevée et elles contribuent à améliorer la radio-opacité du matériau.

Le matériau selon l'invention est mis en forme par moulage, type injection ou extrusion, d'un mélange homogène de constituants. Le matériel et les conditions de moulage sont adaptés à ce mélange, et notamment à la nature du liant utilisé.

Pour conserver un matériau moulable ayant suffisamment de tenue et de résistance, le liant polymère représente 65% à 90 %, en poids du matériau final.

D'autre part, pour apporter suffisamment d'éléments chimiques destinés à favoriser l'intégration biologique, les composants complémentaires (phosphate tricalcique et/ou phosphate dicalcique et/ou hydroxyapatite de calcium, associé(s) éventuellement à au moins un composé de type zéolite ou oxyde par exemple, destiné à améliorer l'électrostaticité et la radio-opacité, et à de l'acide orthophosphorique) représentent entre 10 et 35 % en poids du matériau final.

Un bon compromis, notamment en terme de caractéristiques mécaniques correspond sensiblement à 80 % en poids de liant polymère et 20 % en poids de composant(s) complémentaire(s).

L'invention concerne également le procédé d'obtention de ce matériau selon la revendication 1. Le procédé correspondant consiste: - à mélanger de façon homogène un liant moulable biocompatible avec un ou plusieurs composés réalisant un apport de calcium et de phosphate, - à faire subir au mélange obtenu une opération de moulage, - à effectuer une ou plusieurs opérations de décapage de surface et de décontamination de la pièce moulée, - à conditionner aseptiquement ladite pièce décontaminée.

L'opération de décapage surfacique est avantageusement réalisé au moyen d'au moins un bain dans une solution, en particulier de produit décapant, soumise aux ultrasons.

Les opérations de décapage surfacique et de décontamination s'effectuent par passage de la pièce moulée dans des bains successifs d'acide chlorhydrique ou sulfurique, d'acétone, d'eau oxygénée, d'hypochloride de sodium et de produit(s) désinfectants(s), soumis aux ultrasons, séparés par des opérations de rinçage à l'eau ou de passage dans des bains d'eau soumis aux ultrasons.

### Exemples

Des mélanges de base sont réalisés à partir de poly(étheréthercétone) (PEEK), de phosphate tricalcique (Ca₃ (PO₄)₂), et de dioxyde de titane (TiO₂).

Le PEEK se présente sous la forme d'une poudre ou de granulés (taille : environ 100 microns), distribués par la Société Victrex Europa GmbH, Hauptstr. 11 D-65719 HOFHEIM - Allemagne.

Le phosphate tricalcique est disponible sous forme poudreuse (grains de taille voisine de 200 microns) ; il est par exemple commercialisé par la S.A. Coopération Pharmaceutique Française, 77020 MELUN - France.

L'oxyde de titane est également disponible sous la forme d'une poudre distribuée par la S.A. Coopération Pharmaceutique Française, 77020 MELUN - France ;

### a) Proportions

Il est indiqué ci-après quelques exemples de compositions possibles :

| Mélange 1 (10 % de charges) | | Mélange 2 (20 % de charges) | |
|---|---|---|---|
| - PEEK : | 90 % en poids | - PEEK : | 80 % en poids |
| - Ca₃(PO₄)₂ : | 5 % en poids | - Ca₃(PO₄)₂ : | 10 % en poids |
| - TiO₂ : | 5 % en poids | - TiO₂ : | 10 % en poids |

| Mélange 3 (30 % de charges) | | Mélange 4 | |
|---|---|---|---|
| - PEEK : | 70 % en poids | - PEEK : | 65 % en poids |
| - Ca₃(PO₄)₂ : | 15 % en poids | - Ca₃(PO₄)₂ : | 17,5 % en poids |
| - TiO₂ : | 15 % en poids | - TiO₂ : | 17,5 % en poids |

### b) Malaxage

Les constituants de chaque mélange sont placés dans un mélangeur à turbine jusqu'à obtention d'une parfaite homogénéisation.

### c) Séchage

Chaque mélange homogène obtenu est séché dans une étuve à circulation d'air pendant 3 heures à 150°C.

### d) Moulage

L'opération de moulage est réalisée sur presse à injecter type KRAUSS-MAFFEI, Modèle 90-340-32, Société KRAUSS MAFFEI FRANCE, 92632 GENNEVILLIERS - FRANCE.

Les conditions de préparation du matériel et les conditions de moulage du mélange correspondent à la brochure commerciale « ICI MATERIALS » indiquée ci-avant.

Le PEEK étant un thermoplastique semi-cristallin, il est nécessaire de chauffer le moule à une température au moins supérieure à celle de sa transition vitreuse (140°C). Sinon la qualité de surface des pièces moulées en serait affectée. En effet, le voile de surface serait en phase amorphe et le coeur en phase cristalline ; si le moule était trop froid, les pièces pourraient même avoir un caractère totalement amorphe et les caractéristiques mécaniques chuteraient sévèrement.

La thermorégulation du moule est assurée par un réchauffeur d'huile permettant de le maintenir à une température de l'ordre de 160°C. Des moyens d'isolation limitent les dispersions thermiques et préservent les organes périphériques de la presse à injecter. Ces moyens peuvent se présenter sous la forme de plaques isolantes formées d'un complexe de fibres de verre.

Pour les injections en série, un vibreur sera avantageusement fixé sur la trémie afin de favoriser l'écoulement du mélange.

De façon générale, le moulage est réalisé à une température de l'ordre de 340 à 400°C et à une pression d'injection voisine de 70 à 140 MPa.

Le moule peut être conformé en fonction de la pièce que l'on désire obtenir, par exemple pour la réalisation d'une prothèse osseuse, notamment pour des applications orthopédiques. On peut également obtenir un bloc de matière que l'on va ensuite découper ou usiner selon la forme désirée, pour un comblement osseux ou un implant, type dentaire par exemple .

### e) Décapage surfacique - décontamination

Une fois le matériau moulé obtenu, on le soumet à des opérations de décapage surfacique et de décontamination, avant conditionnement aseptique.

Ces opérations sont avantageusement conduites dans un premier temps par passage du matériau moulé dans différents bains de produits soumis aux ultrasons ; chaque produit utilisé peut jouer un rôle de décapant surfacique ou de désinfectant, ou les deux à la fois.

En tout état de cause, le ou les produits assurant la fonction de décapage surfacique sont adaptés, en liaison avec les ultrasons, de manière à faire apparaître en surface notamment le phosphore et le calcium (sous forme de phosphate de calcium), et le dioxyde de titane. L'accessibilité en surface du phosphate de calcium favorise les échanges avec l'environnement et l'accrochage électrostatique de certains éléments chimiques présents dans l'environnement biologique du matériau après implantation ; ces échanges et/ou ces accrochages électrostatiques induisent une pénétration cellulaire dans le matériau implanté. Egalement, la présence en surface de dioxyde de titane potentialise ces échanges et la présence de liaisons électrostatiques.

En outre, le phosphate de calcium étant au moins partiellement résorbable, sa disparition après implantation du matériau permet la création de cavités ou d'un réseau de cavités, favorisant la pénétration cellulaire des tissus environnants.

Les produits utilisés pour ces opérations de décapage et de décontamination peuvent être l'acide chlorhydrique, (HCl, par exemple à 30 %) ou l'acide sulfurique (H₂SO₄, par exemple à 30 %), l'acétone (C₃H₆O), l'eau oxygénée (H₂O₂, à 110 vol. ou 30 % par exemple), et/ou l'hypochloride de sodium (NaClo) utilisés de préférence en combinaison. Avantageusement, on utilise des bains complémentaires de produits à fonction purement désinfectante, du genre Gigasept (marque déposée) ou Lysetol (marque déposée).

Le protocole correspondant pour mettre en oeuvre ces opérations de décapage/décontamination peut consister à placer l'implant dans les différents bains successifs suivants soumis aux ultrasons :
- HCl 30 % : 20 mn
- H₂O : 10 mn (ou rinçage)
- acétone : 20 mn
- H₂O : 10 mn (ou rinçage)
- H₂O₂ 30 % : 20 mn
- NaclO : 20 mn
- H₂O : 10 mn (ou rinçage)
- Gigasept 12 % : 60 mn
- H2O Ppi : 20 mn (ou rinçage)

L'implant est inséré dans une gaine de stérilisation pour son passage en autoclave ; il subit ensuite un cycle de stérilisation à une température de l'ordre de 135°C pendant 10 minutes, sous une pression de l'ordre de 2150 mbar. Cette opération de stérilisation par autoclave contribue à la fonction de décapage surfacique ; elle peut être associée à un traitement par oxyde d'éthylène ou par rayons gamma.

### f) Résultats

Une analyse par microscope électronique à balayage (MEB) montre que les opérations de décapage/décontamination et de stérilisation favorisent l'apparition des phosphates de calcium en surface. Ces phosphates de calcium émergent par des micropores et cristallisent.

Après implantation, l'analyse de surface montre la présence de trous et de crevasses en surface du matériau, et également la présence de carbone, d'oxygène et d'azote, alors que l'on retrouve peu de calcium et de phosphore en rapport aux concentrations initiales intégrées.

Cela tend à montrer la disparition partielle des particules de phosphate de calcium en surface, et la colonisation des trous et crevasses par des matériaux biologiques environnants, signe d'une acceptation biologique de type greffe.

Une analyse clinique à partir d'implants posés montre que le matériau en question développe à son contact une corticale osseuse conséquence des caractéristiques physiques et atomiques du matériau.

Il s'agit là d'un véritable principe de greffe ; ces résultats démontrent la réalité clinique d'une intégration du matériau au tissu environnant.

## Revendications

1. Procédé d'obtention d'un matériau à usage médical ou vétérinaire, en particulier pour la réalisation d'implants endo-osseux notamment dentaires, ou pour la réalisation de prothèses osseuses, lequel matériau se présente sous la forme d'une pièce moulée, constituée de 65% à 90 % en poids d'un liant polymère biocompatible et, de 10% à 35 % en poids de composant(s) complémentaire(s) en forme d'hydroxyapatite de calcium et/ou de phosphate dicalcique ou tricalcique, réalisant un apport de calcium et de phosphore, associé(s) éventuellement à au moins une zéolite ou un oxyde, lequel procédé consiste :
- à mélanger de façon homogène le liant polymère biocompatible, le ou les composant(s) complémentaire(s) réalisant un apport de calcium et de phosphore, et éventuellement à au moins une zéolite ou un oxyde,
- à faire subir au mélange obtenu une opération de moulage,
- à effectuer des opérations de décapage de surface et de décontamination de la pièce moulée par passage de la pièce moulée dans les bains successifs suivants :
- un bain d'acide chlorhydrique ou sulfurique, soumis aux ultrasons,
- un bain d'acétone, soumis aux ultrasons,
- un bain d'eau oxygénée, soumis aux ultrasons,
- un bain d'hypochloride de sodium, soumis aux ultrasons, et
- un bain de produit(s) désinfectant(s), soumis aux ultrasons,
lesquels bains successifs sont séparés par des opérations de rinçage à l'eau ou de passage dans des bains d'eau soumis aux ultrasons, puis
- à soumettre la pièce moulée à une opération de stérilisation par autoclave, après passage dans lesdits bains de solution soumis aux ultrasons,
- à conditionner aseptiquement ladite pièce décontaminée.

2. Procédé selon la revendication 1, **caractérisé en ce qu'**il comporte un liant en forme de polymère thermoplastique.

3. Procédé selon la revendication 2, **caractérisé en ce qu'**il comporte un liant en forme de polymère thermoplastique du genre PEEK (polyétheréthercétone)

4. Procédé selon l'une quelconque des revendications 1 à 3, **caractérisé en ce qu'**il comporte un liant en forme de polymère naturel du genre cellulose.

5. Procédé selon l'une quelconque des revendications 1 à 4, **caractérisé en ce qu'**il comporte un composé de type zéolite ou oxyde, genre TiO₂, SiO₂, Al₂O₃ ou ZrO₂.

6. Matériau à usage médical ou vétérinaire, en particulier pour la réalisation d'implants endo-osseux notamment dentaires, ou pour la réalisation de prothèses osseuses, susceptible d'être obtenu par le procédé selon l'une quelconque des revendications 1 à 5.

7. Matériau à usage médical ou vétérinaire selon la revendication 6, **caractérisé en ce que** le liant est en forme de polymère thermoplastique du genre PEEK (polyétheréthercétone), et **en ce que** le composé de type zéolite ou oxyde est du genre TiO₂, SiO₂, Al₂O₃ ou ZrO₂.

8. Application du matériau selon l'une quelconque des revendications 6 ou 7, ou obtenu par le procédé selon l'une quelconque des revendications 1 à 5, pour la réalisation in vitro d'implants endo-ossaux, notamment dentaires.

9. Application du matériau selon l'une quelconque des revendications 6 ou 7, ou obtenu par le procédé selon l'une quelconque des revendications 1 à 5, pour la réalisation in vitro de prothèses osseuses.

## Claims

1. Method for obtaining a material for medical or veterinary use, in particular for making endosseous implants, particularly for dental purposes, or for making bone prostheses, said material being in the form of a moulded workpiece, made of 65 % to 90 % by weight of a biocompatible polymer binder, and of 10 % to 35 % by weight of an additional component(s) in the form of calcium hydroxyapatite and/or dicalcium or tricalcium phosphate, achieving a calcium and phosphorus provision, possibly associated with at least one zeolite or oxide, said method consisting in:
- mixing in a homogeneous manner the biocompatible polymer binder, the additional component(s) achieving a calcium and phosphorous provision, and possibly at least one zeolite or oxide,
- subjecting the mixture obtained to a moulding operation,
- performing surface cleaning and decontamination operations of the moulded workpiece by passing said moulded workpiece into the following successive baths:
- a hydrochloric acid or sulphuric acid bath subjected to ultrasonic excitation
- an acetone bath subjected to ultrasonic excitation,
- a hydrogen peroxide bath subjected to ultrasonic excitation,
- a sodium hypochloride bath subjected to ultrasonic excitation, and
- a bath containing a disinfectant product subjected to ultrasonic excitation,
said successive baths being separated by rinsing operations with water or by passing into water baths subjected to ultrasonic excitation, then
- subjecting the moulded workpiece to a sterilisation operation via an autoclave, after passing into said solution baths subjected to ultrasonic excitation,
- packing said decontaminated workpiece in an aseptic manner.

2. Method according to claim 1, **characterised in that** it comprises a binder in the form of a thermoplastic polymer.

3. Method according to claim 2, **characterised in that** it comprises a binder in the form of a thermoplastic polymer such as PEEK (polyetheretherketone).

4. Method according to any one of claims 1 to 3, **characterised in that** it comprises a binder in the form of a natural polymer such as cellulose.

5. Method according to any one of claims 1 to 4, **characterised in that** it comprises a zeolite or oxide-type compound such as TiO₂, SiO₂, Al₂O₃ or ZrO₂.

6. Material for medical or veterinary use, in particular for making endosseous implants, particularly for dental purposes, or for making bone prostheses, capable of being obtained by the method according to any one of claims 1 to 5.

7. Material for medical or veterinary use according to claim 6, **characterised in that** the binder is in the form of a thermoplastic polymer such as PEEK (polyetheretherketone) and **in that** the zeolite or oxide-type compound is for example TiO₂, SiO₂, Al₂O₃ or ZrO₂.

8. Application of the material according to any one of claims 6 or 7, or obtained by the method according to any one of claims 1 to 5, for the in vitro production of endosseous implants, particularly for dental purposes.

9. Application of the material according to any one of claims 6 or 7, or obtained by the method according to any one of claims 1 to 5, for the in vitro production of bone prostheses.

## Patentansprüche

1. Verfahren zur Erlangung eines Materials zur medizinischen oder tiermedizinischen Verwendung, insbesondere zur Herstellung von enossalen Implantaten hauptsächlich zur zahnärztlichen Zwecken, oder zur Herstellung von Knochenprothesen, das genannte Material ist in Form eines Gussteil vorhanden, bestehend aus 65 bis 90 Gewichtprozent eines biologisch verträglichen Polymer Bindungsmittel und, aus 10 bis 35 Gewichtprozent von ergänzende Komponenten in Form von Calciumhydroxylapatit und/oder Dicalciumphosphat oder Tricalciumphosphat, wobei das Material ein Calcium und einen Phosphor Einlass durchführt, eventuell an mindesten einen Zeolith oder ein Oxyd verknüpft, besagtes verfahren besteht darin:
- einheitlich das biologisch verträglichen Polymer Bindungsmittel zu mischen, wobei die ergänzende Komponente(n) ein Calcium und einen Phosphor Einlass besorgt und eventuell an mindesten einen Zeolith oder ein Oxyd,
- an die erhaltene Mischung ein Gießvorgang zu unterziehen,
- oberflächliche Reinigungsvorgänge und Dekontaminierungsvorgänge des Gussteils mittels Eintauchen des Gussteils in die unten stehenden aufeinanderfolgende Bädern, durchzuführen:
- Salzsäurebad oder Schwefelsäurebad mittels Ultraschallen
- Azetonbad mittels Ultraschallen
- Wasserstoffperoxid-Bad mit Ultraschallen
- Natriumhypochlorid-Bad mit Ultraschallen, und
- Desinfektionsmittel-Bad mit Ultraschallen,
die genannten aufeinanderfolgenden Bäder sind durch Spülvorgänge im Wasser voneinander getrennt oder durch Eintauchen in Wasserbäder mit Ultraschallen, dann
- das Gussteil einen Sterilisationsvorgang durch Autoklav nach Eintauchen in die besagten Lösungsbäder mit Ultraschallen zu unterziehen,
- das besagte Teil aseptisch zu verpacken.

2. Verfahren nach Anspruch 1, **dadurch gekennzeichnet, dass** es ein Bindungsmittel in Form von thermoplastischem Polymer aufweist.

3. Verfahren nach Anspruch 2, **dadurch gekennzeichnet, dass** es ein Bindungsmittel in Form von thermoplastischem Polymer in der Art der PEEK (Polyetheretherketon) aufweist.

4. Verfahren nach einem beliebigen der Ansprüche 1 bis 3, **dadurch gekennzeichnet, dass** es ein Bindungsmittel in Form von natürlichem Polymer in der Art der Zellulose aufweist.

5. Verfahren nach einem beliebigen der Ansprüche 1 bis 4, **dadurch gekennzeichnet, dass** es eine Mischung von Zeolithen Typ oder Oxyd Typ in der Art der TiO₂, SiO₂, Al₂O₃ oder ZrO₂ aufweist.

6. Material zur medizinischen oder tiermedizinischen Verwendung, insbesondere zur Herstellung von enossalen Implantaten hauptsächlich zur zahnärztlichen Zwecken, oder zur Herstellung von Knochenprothesen, das nach einem beliebigen der Ansprüche 1 bis 5 durch das Verfahren erlangt werden kann.

7. Material zur medizinischen oder tiermedizinischen Verwendung nach Anspruch 6, **dadurch gekennzeichnet, dass** das Bindungsmittel in Form von thermoplastischem Polymer in der Art der PEEK (Polyetheretherketone) ist, und **dadurch gekennzeichnet, dass** die Mischung von Zeolithen Typ oder Oxyd Typ in der Art der TiO₂, SiO₂, Al₂O₃ oder ZrO₂ ist.

8. Anwendung des Materials nach einem beliebigen der Ansprüche 6 oder 7, oder des erhaltenen Materials durch das Verfahren nach einem der beliebigen der Ansprüche 1 bis 5 für die In-Vitro-Herstellung von enossalen Implantaten hauptsächlich zur zahnärztlichen Zwecken.

9. Anwendung des Materials nach einem beliebigen der Ansprüche 6 oder 7, oder des erhaltenen Materials durch das Verfahren nach einem der beliebigen der Ansprüche 1 bis 5 für die In-Vitro-Herstellung von Knochenprothesen.
